# EUROPEAN PATENT SPECIFICATION

(11) **EP 1 954 676 B1**
(45) Date of publication and mention of the grant of the patent: **07.11.2012**
(21) Application number: 06829080.8
(22) Date of filing: 20.11.2006
(51) Int. Cl.: C07D 211/32

(54) **PROCESS FOR MAKING DONEPEZIL**
VERFAHREN ZUR HERSTELLUNG VON DONEPEZIL
PROCEDE POUR PREPARER LE DONEPEZIL

(30) Priority: 18.11.2005 US 737751 P
(43) Date of publication of application: 13.08.2008
(73) Proprietor: Synthon B.V., 6545 CM Nijmegen (NL)
(72) Inventor: POSPISILIK, Karel, 679 05 Krtiny (CZ)
(74) Representative: van der Sterren-Mol, Josephine
(86) International application number: PCT/EP2006/011129
(87) International publication number: WO 2007/057226

(56) References cited:
- WO-A-97/22584
- DATABASE WPI Week 2005 Derwent Publications Ltd., London, GB; AN 2005-366806 XP002423145 -& WO 2004/044805 A (HEMEI BIOTECHNOLOGY CO LTD TIANJIN) 19 May 2005 (2005-05-19)

## Description

### Background of the Invention

The present invention relates to processes for making donepezil, and to controlling certain impurities.

Donepezil is the common or generic name for the compound 1-benzyl-4-((5,6-dimethoxy-1-indanon)-2-yl)methylpiperidine, which can be represented by formula (I).

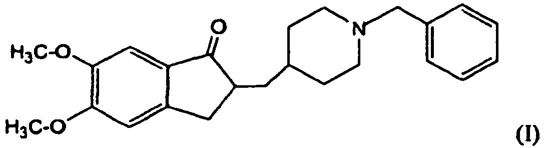

Donepezil is an effective and well-tolerated pharmaceutical for the treatment of Alzheimer disease without liver toxicity. It is a reversible inhibitor of acetylcholinesterase, which is responsible for the breakdown of the neurotransmitter acetylcholine. Donepezil is marketed in the form of tablets containing 5 or 10 mg of donepezil hydrochloride.

Donepezil and its pharmaceutically acceptable salts, e.g., donepezil hydrochloride, were disclosed EP 296560/US 4895841. Various synthetic approaches leading to donepezil have been suggested in the prior art.

### Route A: Via Dehydrodonepezil (III)

The US 4895841 teaches a process wherein 5,6-dimethoxyindan-1-one (IV) and N-benzylpiperidine-4-carbaldehyde (IIa) react to form the indanonylidene precursor of donepezil (Dehydrodonepezil, (III)) which is then reduced to donepezil, advantageously by hydrogen. 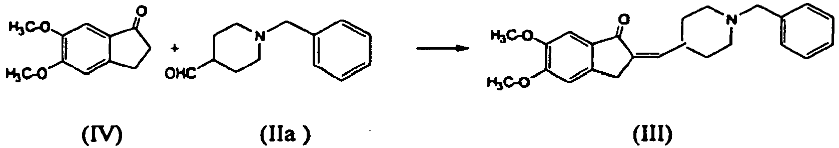 However, the overall yield described in the patent was very low, less than 10%. An improved process for making the dehydrodonepezil (III) was disclosed, however, in JP 11171861. This process comprises reacting (IV) with (IIa) in the presence of metal alkoxides (e.g. MeONa/MeOH in THF).

### Route B: via Desbehzyldonepezil (V).

Donepezil can also be prepared from 4-(5,6-dimethoxyindanon-2-yl-) methylpiperidine ["Desbenzyldonepezil"] (V) by alkylation with benzylbromide, as taught in WO 97/22584. 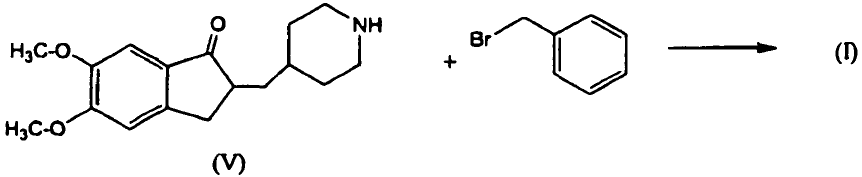 The process for making the compound (V) is generally taught to be from a compound having the general formula (VI) 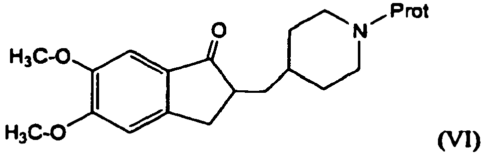 wherein "Prot" is a suitable protective group for the N-atom that can be cleaved to form (V). Advantageously such a protective group is a C1-C4 alkoxycarbonyl, a C1-C4-alkylcarbonyl, or a phenylcarbonyl (e.g., benzoyl) group. But the material (VI) is prepared by a somewhat complicated synthesis involving a cyclization reaction to form the indole ring. Because of this, the whole synthesis is generally even less economical than Route A.

A process for the preparation of the compound (V) is disclosed in WO 2005/003092

### Route C: via Ylidene pyridinium donepezil (VII)

Another synthetic route has been described in EP 711756/US 5606064. The patent suggests that an indanonylidene pyridinium salt of the formula (VII), 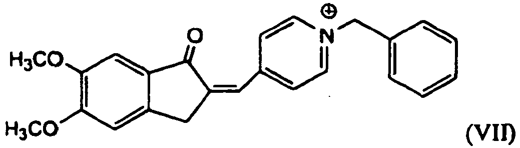 wherein the salt anion is Cl, Br, I, tosylate, or SO₄, is reduced to donepezil by hydrogen in the presence of a hydrogenation catalyst (preferably platinum and its compounds), preferably in an inert solvent.

The starting pyridinium salts (VII) are prepared by condensation of 5,6-dimethoxyindan-1-one (IV) with pyridine-4-carbaldehyde (IIb), followed by N- 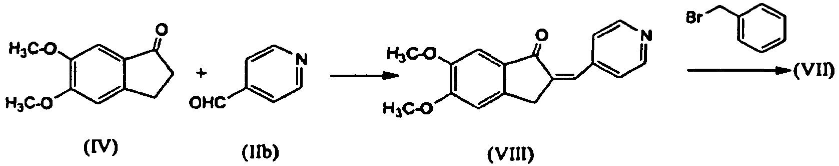 alkylation (quartemization) of the intermediate indanonylidine-pyridine (VIII) by benzylbromide.

The overall yield of this route to donepezil is 58%. In practice, it has been observed that the process suffers from a large amount of side product(s) being formed. Column chromatography was tried to purify the product, but there was one unknown impurity which has the same R_{f} value as donepezil in almost all eluens, so purification was not practically possible. Thus, the process is inconvenient for making donepezil of a high purity, particularly for pharmaceutical purposes.

### Route D: via Pyridiniumdonepezil (IX)

A similar synthetic approach as Route C was described in WO 99/36405. The pyridinium intermediate is however different as it does not contain the double bond attached to the indane nucleus (compound (IX)). 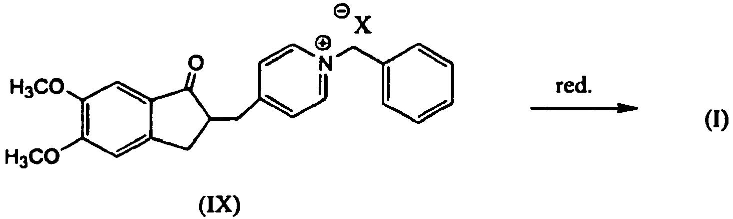 The compound (IX) has been made by the following general scheme: 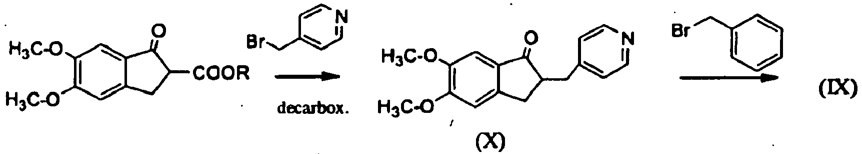 The 5,6-dimethoxy-2- ethoxycarbonyl-1-indanone is treated with NaH and 4-pyridylmethyl bromide to give 5,6-dimethoxy-2-ethoxycarbonyl-2-(4-pyridylmethyl)-1-indanone, which is refluxed with aqueous ethanol containing KOH to give 5,6-dimethoxy-2-(4-pyridylmethyl)-1-indanone (X) upon decarboxylation. The latter compound is treated by benzylbromide to give 1-benzyl-4-[(5,6-dimethoxy-1-oxoindan-2-yl)methyl]pyridinium bromide (compound (IX), X = Br).

The process suffers from the same disadvantages as cited for Route C, namely the production of impurities and the difficulty in practically purifying the desired donepezil.

WO 2005/044805 (XP 002423145) discloses a process for preparing donepezil by hydrogenation of an acid addition salt of compound (VIII) in the presence of PTO² and the subsequent reaction with benzyl bromide.

In J. Pharm. Biomed. Anal. (2004) 35, 1047-1058 by K.V.S.R. Krishna Reddy *et al*., several potential impurities in the process to prepare donepezil were identified and characterized.

It would be desirable to find a process/synthetic route for making donepezil in good yield. It would also be desirable to have a route whereby donepezil can be obtained in high purity.

### Summary of the Invention

The present invention is based on the discovery of a new route for making donepezil.

A first aspect of the invention relates to a process for making donepezil, which comprises:
(a) hydrogenating an acid addition salt of formula (VIIIa) 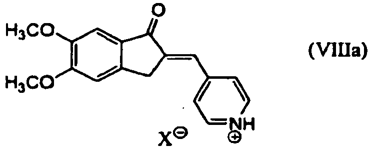 wherein X represents an acid counter ion, by reaction with hydrogen in the presence of a hydrogenation catalyst to form a compound of formula (V) 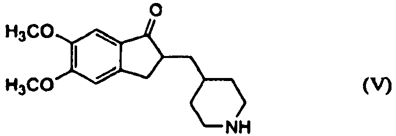 wherein said hydrogenation step (a) is carried out until the amount of the partial hydrogenation product represented by formula (X) 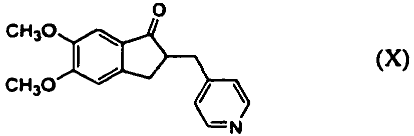 is 0.5% or less of the amount of the compound of formula (V);
   and
(b) reacting said compound of formula (V) with a benzylhalide to form donepezil of formula (I) 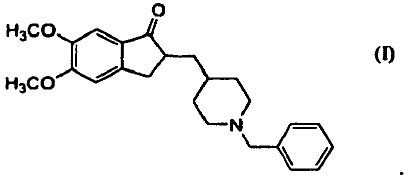 In this way the compound (V) can be more economically formed than in Route (B) of the prior art. In some embodiments, the hydrogenation reaction can be performed in an aqueous environment and using cheaper catalysts such as palladium-based catalysts. Also, compound (V) can be formed in high purity which can facilitate the formation and/or isolation of donepezil in high purity. In a preferred embodiment, the compound (V) is produced such that the amount of the compound (X) is 0.5% or less and the amount of compound (XI) is not more than 5.0% 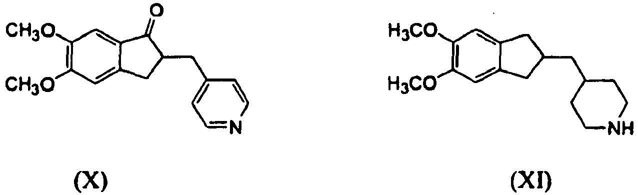 of the amount of compound (V).

According to a preferred embodiment of the present invention relates to process further comprises:
assaying said donepezil or donepezil-containing solution for the presence of a compound of formula (XII) 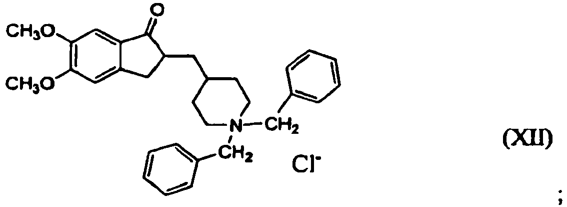 and
   if said compound of formula (XII) is present in an amount greater than a predetermined level, then separating said compound of formula (XII) from said donepezil by forming a donepezil solution in ethyl acetate. The compound (XII) is a novel compound that can be formed when benzylchloride is used in the alkylation step to form donepezil. Surprisingly compound (XII) is insoluble in ethyl acetate whereas donepezil is sufficiently soluble in ethyl acetate so as to allow for a separation/extraction of the donepezil.

### Detailed Description of the Invention

As used herein, the word "isolated" refers to separating the target compound from at least a portion of its environment so as to recover the target compound in a more concentrated form. Typically the isolation step involves a phase separation technique wherein the target compound is preferentially obtained in one phase whereby it is more easily recovered in a more concentrated form. Traditional examples of isolation techniques include precipitation and/or crystallization (e.g., solid-liquid separations), evaporating or distilling off all or a portion of the solvent(s) (e.g., vapor-liquid separations), liquid-liquid phase separations such as by extractions or decanting, etc. While isolation can and frequently does have a purification effect, it is not required that impurities *per se* are reduced or removed.

In general, the overall synthetic scheme of the present invention can be represented by the following sequence:

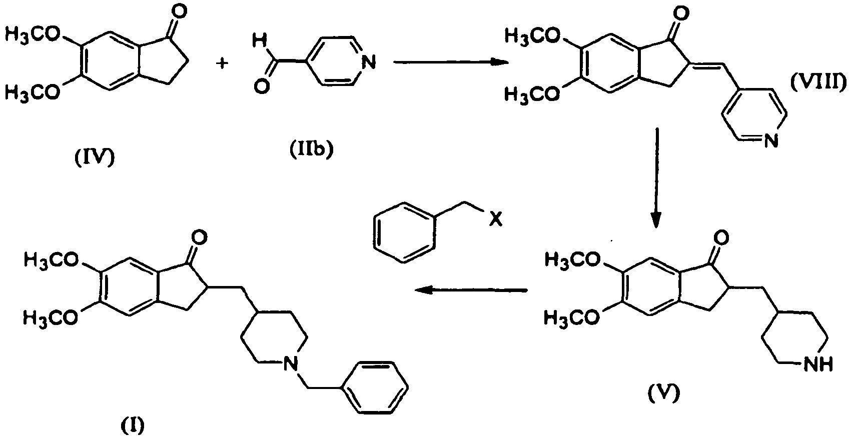

Significantly, the pyridine and olefin double bond moieties are hydrogenated or reduced before the alkylation step. That is, the reduction or hydrogenation step forms a compound (V) and not donepezil (I). However, in order for an efficient reduction reaction, it is important that the pyridine ring in compound (VIII) be protonated, otherwise the reduction of the pyridine ring would be too slow. While the prior art routes C) and D) fulfilled this general condition by means of a quartemization reaction, i.e. alkylating with a benzylhalide reagent before reduction, the present process uses a simpler way of protonating the compound (VIII); i.e., forming an acid addition salt of formula (VIIIa): 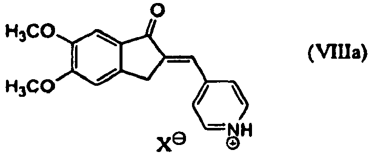 wherein X is an acid counter ion. For clarity, an "acid counter ion" refers to the counter ion formed from (or the residue of) the acid used in making the acid addition salt. The acid is not particularly limited and includes organic and inorganic acids such as HCl, HBr, sulfonic acids including methane sulfonic acid, toluene sulfonic acid, etc. A preferred salt of formula (VIIIa) is, for reasons explained hereinafter, a tosylate salt (i.e., formed from toluene sulfonic acid) represented by formula (VIIIb):

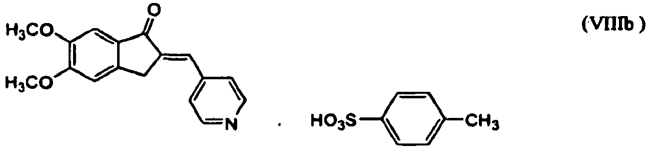

In any event, it is advantageous that the salt compound (VIIIa) is charged into the hydrogenation reaction mixture as an acid addition salt, i.e. having the pyridine ring adequately protonated. There is no need to use an acid as a solvent or to add an acid into the reaction mixture, though such possibilities are not excluded. Further, by using the acid addition salt compound (VIIIa), it is possible to perform the hydrogenation reaction in an aqueous environment, e.g. the salt in an aqueous slurry/suspension and/or in solution. Preferably the acid addition salt of formula (VIIIa) is completely dissolved in the water solvent. Because an aqueous environment can be used, it is possible to use economically advantageous hydrogenation catalysts, for instance palladium catalysts. In this embodiment, the catalysts typically comprise 3 to 10 % palladium on charcoal.

The compounds of formula (VIII) can be formed by methods known *per se.* For example, the compounds can be formed by the condensation of 5,6-dimethoxyindan-1-one (IV) with a slight excess of pyridine-4-carbaldehyde (IIb), in a solvent under the presence of an acid, followed by neutralization of the acid by a base, as described in EP 711756. To form the acid addition salt of formula (VIIIa), several variants of the basic synthesis can be used. In one variant, the condensation of compound (IV) with the compound (IIb) is not followed by the neutralization step. Instead, the *in situ* formed salt of the compound (VIII) is directly used in the next step, preferably after its isolation from the reaction mixture. In a second variant, the isolated base of the compound (VIII) prepared according to the known process is converted into an acid addition salt in a separate step. The first variant is technically simpler and is normally preferred. In accordance with the teaching of EP 711756, the compound (IV) reacts with the compound (IIb) in an inert solvent such as toluene, preferably in the presence of a p-toluenesulfonic acid. Thus, the tosylate salt of formula (VIIIb) can be directly isolated.

The tosylate salt is a particularly advantageous intermediate because it is a stable crystalline compound that can be safely stored and purified if necessary. In particular, the tosylate salt (VIIIb) is the preferred one among acid addition salts of the compound (VIII) in that:
a) It is a stable solid material, which may be safely stored for a prolonged period, it is not an irritant and is non-hygroscopic; and
b) It is well soluble in hot water, therefore, it may be used in the hydrogenation reaction step in an aqueous solution.

The tosylate salt may be, in further, simply purified by charcoal treatment and crystallization from water. The aim of purification is to remove components poisoning the hydrogenation catalyst in the following step.

Regardless of how it is formed, the acid addition salt (VIIIa) can be reduced to the compound (V) by a hydrogenation reaction. The hydrogenation reaction proceeds by reacting the compound (VIIIa) with hydrogen in the presence of a hydrogenation catalyst. Typically the hydrogenation reaction is carried out in a solvent, especially an aqueous solvent. As used herein, the bubbling of hydrogen gas through (or passing it thereover) an aqueous solution of the acid addition salt of (VIIIa) in the presence of a hydrogenation catalyst is considered to be carrying out the reaction in the solvent, even though three phases may be present when the reactant (VIIIa) is transformed into product (V) within the solvent; i.e., dissolved therein. Suitable hydrogenation reaction conditions including temperatures and pressures as well as suitable catalysts are generally known in the art and/or readily determined by workers skilled in the art using routine skill. Generally the hydrogenation catalysts are noble metals such as platinum, platinum oxide, palladium, rhodium, or ruthenium, optionally supported on a material such as carbon, e.g. charcoal, calcium carbonate, etc. A palladium catalyst in combination with water as the solvent is the preferred arrangement for the hydrogenation process of the present invention. The hydrogenation is preferably performed under enhanced pressure of hydrogen, i.e. in the pressure vessel, and at enhanced temperature, i.e. higher than 25°C.

The hydrogenation of the indanonylindine pyridine compound (VIII) into (V) is a result of two hydrogenation reactions, the first one being the saturation of the olefin double bound which results in the partial hydrogenation product represented by formula (X) followed by slow hydrogenation of the pyridine ring. Over-reduction of the piperidineindanone product (V) may also occur, leading to the over-hydrogenation product (a piperidineindane) of formula (XI). 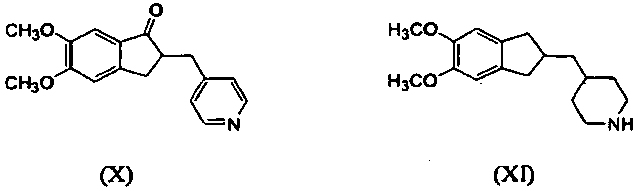 It should be understood that the formulas (V), (X), and (XI) include the protonated and/or salt forms thereof. Thus formula (X) and (XI) cover both the salt form that is likely produced in the hydrogenation reaction as well as the unprotonated base form that is likely measured via, e.g., HPLC, etc.

It was discovered that the hydrogenation intermediate - compound (X) - has very similar solubility properties as the product (V) and undergoes the benzylation reaction in the next step. Therefore, the partial hydrogenation product (X) and/or its benzylated analogue have a strong tendency to penetrate into the final donepezil and it is very difficult to remove it/them from donepezil by conventional crystallization or chromatographic purification methods. It was found that the relative content of (X) in the produced intermediate (V) should not exceed 0.5%, preferably 0.3 % based on the amount of (V). Thus, it is desirable to assure sufficiently strong reaction conditions and to proceed with the hydrogenation reaction until the content of the intermediate drops below 0.5 or 0.3%.

On the other hand, the over-hydrogenation product (XI) may be removed from the final product more easily. Nonetheless, if it is present in the reaction mixture in higher amounts, the economy of the whole process decreases as desired product is lost by performing the necessary purification processes. It was found that a sufficiently pure donepezil may be prepared, without significant waste in purifying, if the relative content of the compound (XI) in the hydrogenation product (V) is lower than 5 %, preferably lower than 2.5 %.

These two requirements are somewhat contradictory. On one hand, the hydrogenation reaction must proceed under sufficiently robust reaction conditions (pressure, temperature and time) to minimize the content of the partial hydrogenation product (X), and, on the other hand, the reaction conditions must be sufficiently mild to avoid the over-hydrogenation product (XI). Put differently, in general as the hydrogenation reaction is carried out, the amount of the partial hydrogenation product (X) gradually becomes smaller while the amount of the over-hydrogenation product (XI) gradually becomes larger. The present invention seeks to balance these competing interests by carrying out the hydrogenation reaction under conditions (temperature, pressure, duration, catalyst, etc.) that produce 0.5 % or less of the partial hydrogenation product (X) as described above. Preferably the hydrogenation reaction is carried out such that the amount of over-hydrogenation product (XI) is also within its above-described range of 5 % or less. A product (V) that meets both of these criteria is considered to be "substantially pure." Substantially pure (V) can be readily converted into relatively pure donepezil in the next step without excessive product loss to purification.

While it is possible to carry out the hydrogenation reaction until the amount of partial hydrogenation product (X) and optionally the amount of over-hydrogenation product (XI) are within the desired amounts using previously determined conditions suitable for achieving such a result, it may nonetheless be advisable to determine the amount of (X) and/or (XI) by monitoring the hydrogenation reaction. Hydrogenation catalysts can have different catalytic power, even when a chemically identical catalyst is used. Catalysts from different companies, and even different batches of the catalyst of the same company, may require an adjustment of the reaction conditions. Therefore, it may be desirable to monitor the hydrogenation reaction step while it is in progress for the presence of the compounds (X) and/or (XI) and to adjust the reaction conditions accordingly. Suitable methods for the monitoring include chromatographic methods such as TLC or HPLC. While each run can be monitored and controlled, it is also possible to simply test the quality of the catalyst and/or conditions by a trial experiment, whereby optimal conditions, particularly pressure of hydrogen, hydrogenation temperature and hydrogenation time, are found to completely convert the pyridineindanone (X) into the desired piperidineindanone (V), but to keep subsequent over-hydrogenation of the piperidineindanone at an acceptable level. The checked reaction conditions may then be used in the full scale production with or without monitoring. In any event, whether because the reaction is monitored or not, if the hygrogenation is stopped and the above impurity conditions are met, then the process is considered to meet the present invention.

After the hydrogenation reaction is terminated, the catalyst is removed by any conventional way, e.g. by filtration, and the reaction product (V), in a substantially pure quality, is advantageously isolated from the reaction mixture. One way of isolation comprises neutralizing the reaction mixture by a suitable base, followed by the precipitation of the product (V) from the reaction mixture. The precipitation may be either direct, or, in a preferred variant, the compound (V) is first extracted from the aqueous environment by an organic solvent sparingly miscible with water, for instance by 1-butanol or with ethyl acetate, and the product is then precipitated from the organic solvent. Typically, the precipitation from a concentrated solution in 1-butanol is performed by mixing said solution with a contrasolvent, which typically is an etheral solvent such as methyl-tert. butyl ether. The precipitated product is isolated by a conventional way, e.g. by filtration or centrifugation, and may be washed and dried.

Optionally, the isolated product may be further purified, e.g. by a crystallization from a suitable solvent, but generally this is not required should the compound (V) meet the following criteria (HPLC): Content of piperidineindanone (V) more than 97.0 % rel., content of piperidineindane (XI) impurity less than 1.0 % rel, content of pyridineindanone (X) impurity less than 0.30 % rel.

In the next step the compound (V) is converted into crude donepezil by an alkylation reaction with a benzylhalide, preferably by benzylchloride, in a suitable solvent, preferably in toluene. Released hydrogen chloride is neutralized with a suitable base, for instance by sodium hydrogencarbonate.

The principal side reaction in this synthesis step is a subsequent alkylation (quarternization) of donepezil with the benzylhalide, e.g. benzylchloride to form benzyldonepezilium salt, e.g. chloride (XII).

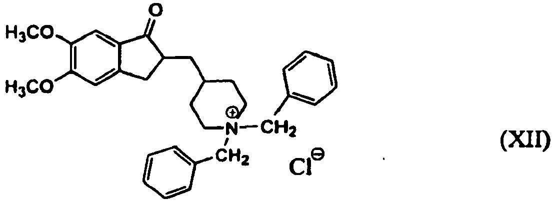

The corresponding bromide salt results from the use of benzylbromide, etc. The higher the excess of benzylchloride, the more the impurity (XII) is formed. On the other hand, a slight excess of the benzylchloride is desirable, to assure the complete conversion of compound (V) into donepezil. Therefore, it would be ideal to know the exact content of the compound (V) in the starting material and to adjust the amount of benzyl chloride accordingly, so that only a small molar excess, i.e., 1 - 10%, is used.

However, even at optimal conditions, about 4 - 7 % (rel.) of the benzyldonepezil impurity (XII) is formed during the alkylation reaction. Fortunately, it was discovered that this impurity is, contrary to the donepezil itself, practically insoluble in low polar solvents, e.g. in ethyl acetate. Thus, donepezil can be purified by preferentially extracting it (dissolving it) in a low polar solvent such as ethyl acetate. The benzyldonepezil impurity being relatively insoluble in ethyl acetate, is left behind, typically as a solid, oil, or other residue. Generally the ethyl acetate is slowly added to a donepezil reaction residue, e.g., to the remainder after evaporating off the reaction solvent, to achieve good purification/extraction. Although the benzyldonepezil removal is efficient, it may be useful to monitor the ethyl acetate solution of donepezil to determine the benzyldonepezil content prior to further elaboration and to repeat the purification step, if needed. By analogy the other possible halide salts of benzyldonepezil are also expected to be removed/separated from donepezil by the use of ethyl acetate.

Furthermore, it is advantageous that the ethyl acetate solution of donepezil is treated with an activated charcoal. This way, further impurities of a nonpolar nature (particularly dimers) may be removed. The quality of the ethyl acetate solution of donepezil, particularly the content of donepezil and the impurities (X), (XI), (XII) and dimers, may be advantageously monitored by HPLC.

The product of the above synthesis and purification is an ethyl acetate solution of donepezil base. In particular, the solution comprises essentially pure donepezil base, i.e. the content of any of the above impurities is less than 0.3 %, and preferably less than 0.1 %, relatively to the content of donepezil.

The ethyl acetate solution of essentially pure donepezil base is a very suitable starting material for making pure, particularly pharmaccutically pure, solid state donepezil and/or its pharmaceutically acceptable salts. From this solution, one may produce the solid state donepezil base and/or any donepezil salt, for instance donepezil hydrochloride, by application of known methods.

### EXAMPLES

### Example 1

### Preparation of 5,6-dimethoxy-2-[1-(4-pyridinyl)methylidene]-1-indanone tosylate (Pyridineindanone)

### 1.1 Preparation of raw product

A 6 L glass reactor equipped with a mechanical stirrer, a reflux condenser with Dean-Starck water trap and oil bath is charged with 1.8 L of toluene. Under vigorous stirring, 0.257 kg of 5,6-dimethoxyindan-1-one is added. Then 0.153 L of pyridine-4-carbaldehyde is added in one portion, followed by addition of 0.296 kg of p-toluenesulfonic acid monohydrate. The mixture is heated to boiling under vigorous stirring and the water formed is azeotropically removed. After all water is removed (approx. 6 hours), the heating is stopped and the reaction mixture is slowly cooled under stirring. The solid material is filtered off, filter cake is rinsed with 0.05 L toluene and dried to give 0.66 kg of raw product.

### 1.2 Purification of raw product

A 6 L glass reactor equipped with a mechanical stirrer, a reflux condenser, and oil bath is charged with 3.8 L of demi-water and 0.66 kg of the raw product from the first step. The mixture is heated up to dissolution (80-90°C) into a brown solution.
Then a mixture of 0.05 kg of activated charcoal in 0.20 L demi-water is added and the mixture is heated under reflux for 30 minutes. After that the mixture is hot filtered through a Cellite layer, the cake is rinsed with 0.10 L boiling hot water, the yellowish filtrate is poured into another bottle and slowly cooled under stirring to ambient temperature and then stirred for 4 hours at 0-5 C. The solid is filtered, the filter cake is rinsed with 0.50 L of cold demi-water and with 0.20 L acetone. The cake is dried to give 0.61 kg of purified 5,6-dimethoxy-2-[1-(4-pyridinyl)methylidene]-1-indanone tosylate.

### Example 2

### Preparation of 5,6-dimethoxy-2-(4-piperidinylmethyl)-1-indanone (Piperidineindanone)

A 20 L pressure vessel is charged with 10 L of demi-water and heated to 60 °C, then a suspension of 0.617 kg pyridineindanone tosylate in 4.0 L of demi-water is added. The content of the vessel is heated further to 70°C. Then, 0.062 kg of 10 % Pd/C catalyst (Degussa E101) mixed with 1.42 L of demi-water is added, the vessel is swept with nitrogen, the temperature of the reaction mixture is increased to 95°C, and the autoclave is swept three times with hydrogen. The reaction mixture is hydrogenated at 10 bar for 8 hours at this temperature upon HPLC control. Then the reaction mixture is cooled to 20 °C, the catalyst is filtered off and washed with 3.0 L demi-water. The colourless filtrate is saturated with 2.50 kg of sodium chloride and a solution of 0.36 kg of sodium hydroxide in 0.70L of demi-water is added. The mixture is three times extracted with 2.0 L of 1-butanol. Combined butanol extracts are evaporated to give 0.42 kg of residue. The residue is slowly poured into a 1.5 L flask equipped with mechanic stirrer charged with 0.70 L of methyl-tert. butyl ether. The precipitated solid is stirred at ambient temperature for 3 hours and is left to stand overnight. Then the solid is filtered off and washed with 0.10L of methyl-tert. butyl ether, the cake is air dried to give 306 g of 5,6-dimethoxy-2-(4-piperidinylmethyl)-1-indanone (Piperidineindanone)

HPLC assay:
Piperidineindanone 97.8 % rel.,
Piperidineindane impurity 0.13 % rel. ,
Pyridineindanone impurity 0.30 % rel.

### Example 3

### 3.1 Preparation of Donepezil free base

A 1.5 L reaction bottle equipped with a stirrer, a thermometer, a dropping funnel, a heating bath and a reflux condenser is charged with 0.71 L toluene, 0.057 kg 5,6-dimethoxy-2-(4-piperidinylmethyl)-1-indanone (Piperidineindanone) and 0.021 kg sodium bicarbonate. The reaction mixture is stirred and preheated to the boiling temperature and then 0.0285 L benzylchloride is carefully dropwise added and the mixture is heated with stirring for 8 hours. The temperature of the bath is set at 145°C. The reaction mixture is analysed by HPLC after 8 hours of reaction. The reaction mixture is cooled and filtered through paper filter. The filter cake is rinsed with 0.280 L toluene. The filtrate is placed into 6 L bottle and washed four times with 1.90 L demi-water, the phases are separated, and the toluene phase is evaporated in vacuo to give 58.0 g of residue.

A 1 L distillation flask, equipped with a magnetic stirrer is charged with 58.0g of the residue and 0.47 L ethyl acetate. The ethyl acetate is added continuously at 65°C dropwise over four hours. The mixture is left stirring until the next day at laboratory temperature and then filtered. The filter cake is rinsed with 0.19 L Ethylacetate. The filtrate is stirred with 0.01 kg active charcoal for 30 minutes and filtered again. Filtrate is analysed by HPLC. 0.68 L of donepezil free base solution was received. HPLC analysis of the ethyl acetate solution: 99.67% donepezil, content of pyridineindanone (rel.) 0.27 %

### 3.2 Preparation of Donepezil hydrochloride monohydrate

A 3 L three necked flask equipped with a mechanic stirrer is charged with 0.68 L ethyl acetate solution of donepezil free base, ethyl acetate is distilled off to get 400 mL of distillation residue. Then the distillation residue is cooled to - 8 °C. Then 8.8 mL hydrochloric acid is dissolved in 58.5 mL methanol and the solution is cooled to - 10°C. This solution is added to the stirred solution of donepezil in ethyl acetate. No exothermic effect is observed. After 2 minutes of stirring , the solution is seeded with 25 mg donepezil hydrochloride monohydrate (Form I). Crystallization started, a small exothermic effect is observed (temperature raises to -6 °C). The resulting suspension is stirred at -6 to -8 °C for 45 minutes. The solid is isolated by filtration on a1L glass-filter which is cooled at 4°C. The wet cake is washed with cold 52 mL ethyl acetate (0 °C) and twice with 35 mL cold n-heptane. The wet solid is spread out on a glass dish and allowed to dry at ambient conditions and mixed regularly for 24 hours. Yield : 40.8 g of donepezil hydrochloride monohydrate.

XRPD showed pure FORM I (monohydrate).

Assay by HPLC:
Donepezil more than 99.6 %
Benzyldonepezil below 0.03 %
Pyridineindanone below 0.06 %
Piperidineindane below 0.03 %

## Claims

1. A process for making donepezil, which comprises:
(a) hydrogenating an acid addition salt of formula (VIIIa) 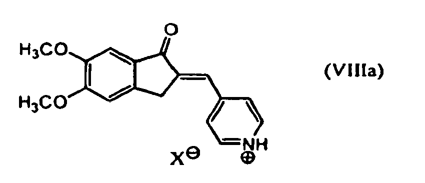 wherein X represents an acid counter ion, by reaction with hydrogen in the presence of a hydrogenation catalyst to form a compound of formula (V) 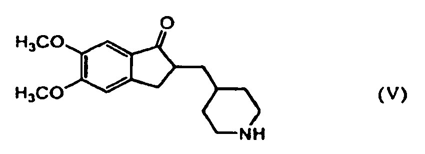 wherein said hydrogenation step (a) is carried out until the amount of the partial hydrogenation product represented by formula (X) 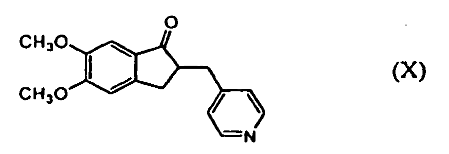 is 0.5% or less of the amount of the compound of formula (V);
and
(b) reacting said compound of formula (V) with a benzylhalide to form donepezil of formula (I) 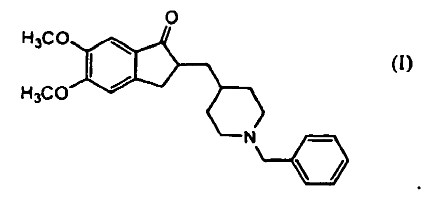 Optionally purifying a donepezil containing solution to obtain donepezil.

2. The process according to claim 1, wherein said hydrogenation step (a) is carried out in an aqueous solvent.

3. The process according to claim 1 or 2, wherein the amount of said partial hydrogenation product represented by formula (X) is determined by monitoring the progress of said hydrogenating step (a).

4. The process according to claim 1-3, wherein X in formula (VIIIa) represents the residue of toluene sulfonic acid.

5. The process according to claim 1-4, wherein said hydrogenation catalyst comprises palladium supported on carbon.

6. The process according to claim 1-5, wherein said hydrogenation reaction step (a) is stopped when the following two criteria are satisfied:
(i) the amount of the partial hydrogenation product represented by formula (X) 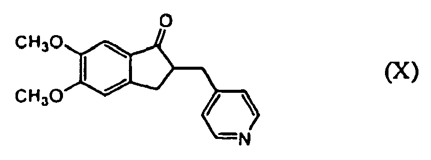 is 0.5% or less of the amount of the compound of formula (V); and
(ii) the amount of the over-hydrogenation product represented by formula (XI) 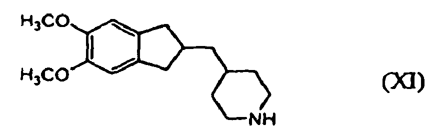 is not more than 5.0% of the amount of the compound of formula (V).

7. The process according to claim 6, wherein satisfaction of said two criteria is determined by monitoring the progress of said hydrogenation step (a).

8. The process according to claim 1-7, which further comprises isolating and optionally purifying said compound of formula (V) before carrying out said reaction step (b).

9. The process according to claim 1-8, wherein said benzylhalide is benzylchloride.

10. The process according to claim 1-9, which further comprises isolating said donepezil of formula (I) via ethyl acetate extraction.

11. The process according to claim 1-10, which further comprises reacting said donepezil of formula (I) with a pharmaceutically acceptable acid to form an acid addition salt of donepezil.

12. The process according to claim 1-11 further comprises:
assaying said donepezil or donepezil-containing solution for the presence of a compound of formula (XII) 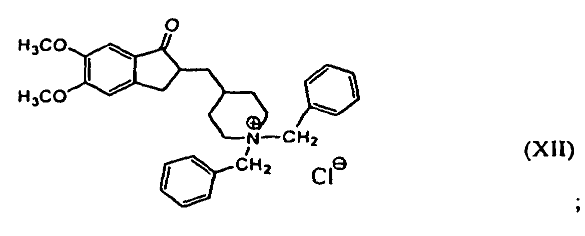 and
if said compound of formula (XII) is present in an amount greater than a predetermined level, then separating said compound of formula (XII) from said donepezil by extracting donepezil in ethyl acetate.

## Patentansprüche

1. Verfahren zum Herstellen von Donepezil, welches umfasst:
(a) Hydrieren eines Säureadditionssalzes der Formel (VIIIa) 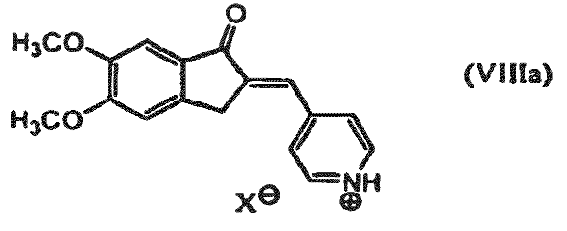 wobei X ein Säuregegenion darstellt, durch Reaktion mit Wasserstoff in der Gegenwart eines Hydrierungskatalysators, um eine Verbindung der Formel (V) zu bilden 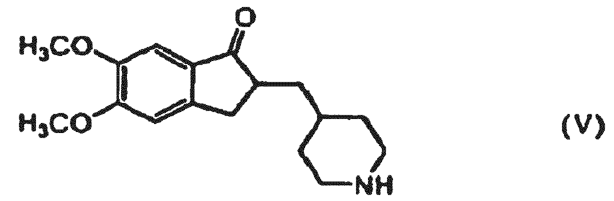 wobei der Hydrierungsschritt (a) durchgeführt wird, bis die Menge des Teilhydrierungsprodukts, dargestellt durch Formel (X) 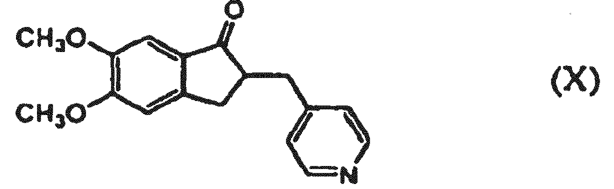 0,5% oder weniger der Menge der Verbindung der Formel (V) beträgt;
und
(b) Reagieren der Verbindung der Formel (V) mit einem Benzylhalogenid, um Donepezil der Formel (I) 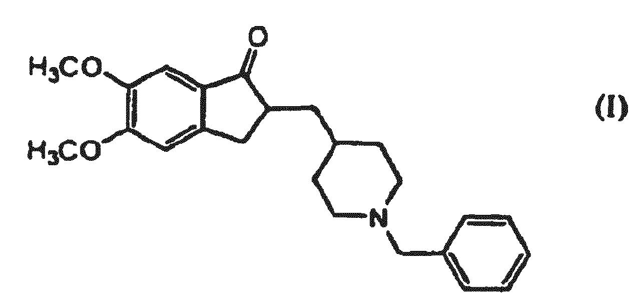 zu bilden,
gegebenenfalls Reinigen einer Donepezil enthaltenden Lösung, um Donepezil zu erhalten.

2. Verfahren nach Anspruch 1, wobei der Hydrierungsschritt (a) in einem wässrigen Lösungsmitten durchgeführt wird.

3. Verfahren nach Anspruch 1 oder 2, wobei die Menge des durch (X) dargestellten Teilhydrierungsprodukts durch Beobachten des Fortgangs des Hydrierungsschritts (a) bestimmt wird.

4. Verfahren nach Anspruch 1-3, wobei X in Formel (VIIIa) den Rest Toluolsulfonsäure darstellt.

5. Verfahren nach Anspruch 1-4, wobei der Hydrierungskatalysator auf Kohlenstoff geträgertes Palladium umfasst.

6. Verfahren nach Anspruch 1-5, wobei der Hydrierungsreaktionsschritt (a) gestoppt wird, wenn die folgenden zwei Kriterien erfüllt sind:
(i) die Menge des Teilhydrierungsprodukts, dargestellt durch Formel (X) 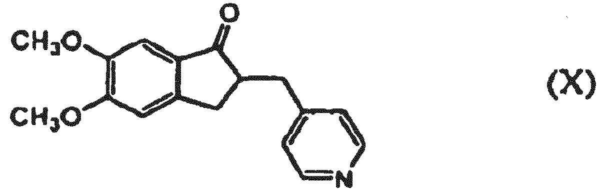 0,5% oder weniger der Menge der Verbindung der Formel (V) beträgt;
und
(ii) die Menge des Überhydrierungsprodukts, dargestellt durch Formel (XI) 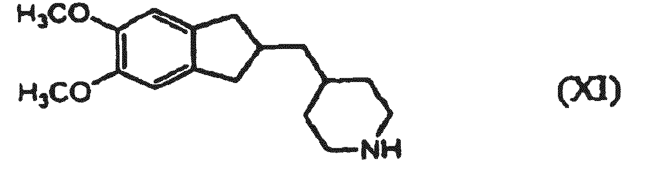 nicht mehr als 5,0% der Menge der Verbindung der Formel (V) beträgt.

7. Verfahren nach Anspruch 6, wobei die Erfüllung der zwei Kriterien durch Beobachten des Fortgangs des Hydrierungsschritts (a) bestimmt wird.

8. Verfahren nach Anspruch 1-7, das des Weiteren Isolieren und gegebenenfalls Reinigen der Verbindung der Formel (V) vor dem Durchführen des Reaktionsschritts (b) umfasst.

9. Verfahren nach Anspruch 1-8, wobei das Benzylhalogenid Benzylchlorid ist.

10. Verfahren nach Anspruch 1-9, das des Weiteren Isolieren des Donepezil der Formel (I) mittels Ethylacetatextraktion umfasst.

11. Verfahren nach Anspruch 1-10, das des Weiteren Reagieren des Donepezil der Formel (I) mit einer pharmazeutisch verträglichen Säure umfasst, um ein Säureadditionssalz von Donepezil zu bildern.

12. Verfahren nach Anspruch 1-11 umfasst des Weiteren:
Testen des Donepezil oder der Donepezil enthaltenden Lösung auf die Gegenwart einer Verbindung der Formel (XII) 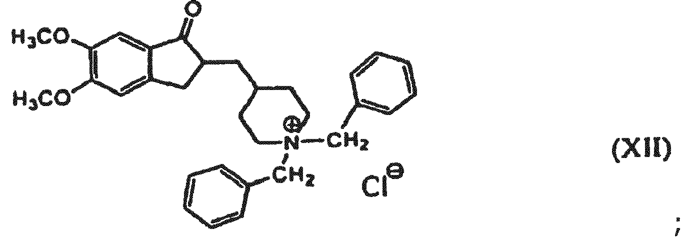 und
falls die Verbindung der Formel (XII) in einer Menge vorhanden ist, die größer ist als eine vorbestimmte Menge, dann Abtrennen der Verbindung der Formel (XII) von dem Donepezil durch Extrahieren von Donepezil in Ethylacetat.

## Revendications

1. Un procédé pour fabriquer du donepezil, qui comprend;
(a) l'hydrogénation d'un sel d'addition d'acide de formule (VIIIa) 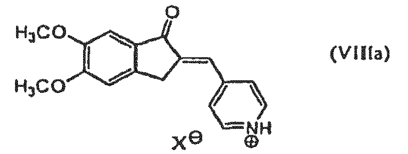 dans laquelle X représente un contre ion acide,
par réaction avec de l'hydrogène en présence d'un catalyseur d'hydrogénation pour former un composé de formule (V) 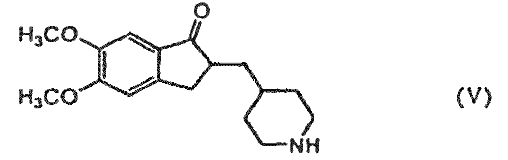 procédé dans lequel ladite d'hydrogénation (a) se poursuit jusqu'à ce que la quantité du produit de l'hydrogénation partielle représenté par la formule (X) 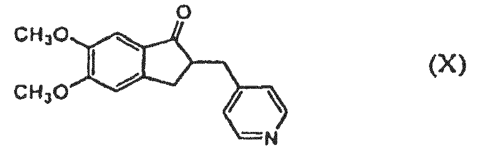 est égale ou inférieure à 0,5% de la quantité de composé de formule (V);
et
(b) réaction dudit composé de formule (V) avec un halogénure de benzyle pour former le donepezil de formule (I) 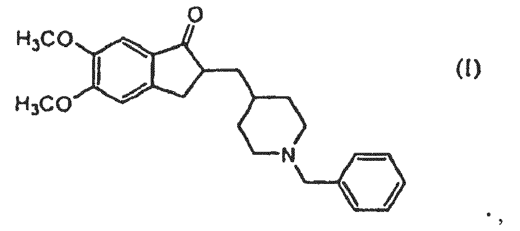 puis purification éventuelle d'une solution contenant du donepezil pour obtenir le donepezil.

2. Le procédé selon la revendication 1, dans lequel ladite étape d'hydrogénation (a) est effectuée dans un solvant aqueux.

3. Le procédé selon la revendication 1 ou 2, dans lequel la quantité dudit produit de l'hydrogénation partielle représenté par la formule (X) est déterminée par contrôle du progrès de ladite étape d'hydrogénation (a).

4. Le procédé selon les revendications 1 à 3, dans lequel X dans la formule (VIIIa) représente le résidu d'acide toluène sulfonique.

5. Le procédé selon les revendications 1 à 4, dans lequel le catalyseur d'hydrogénation comprend du palladium supporté sur du charbon.

6. Le procédé selon les revendications 1 à 5, dans lequel ladite réaction d'hydrogénation (a) est arrêtée lorsque les deux critères suivants sont satisfaits;
(i) la quantité du produit de l'hydrogénation partielle représenté par la formule (X); 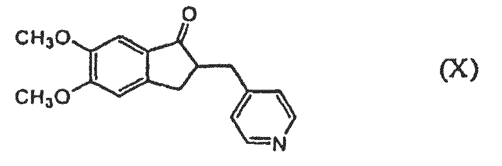 est égale ou inférieure à 0,5% de la quantité du composé de formule (V); et (ii) la quantité du produit excédent de l'hydrogénation représentée par la formule (XI) 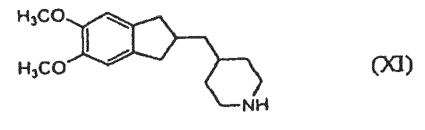 n'est pas supérieure à 5,0% de la quantité de composé de formule (V).

7. Le procédé selon la revendication 6, dans lequel la satisfaction des deux critères précités est déterminée par contrôle du progrès de ladite étape d'hydrogénation (a).

8. Le procédé selon les revendications 1 à 7, comprenant en outre l'isolement et éventuellement la purification dudit composé de formule (V) avant de mettre en oeuvre ladite étape réactionnelle (b).

9. Le procédé selon les revendications 1 à 8, dans lequel ledit halogénure de benzyle est le chlorure benzylique.

10. Le procédé selon les revendications 1 à 9, comprenant en outre l'isolement dudit donepezil de formule (I) par extraction à l'aide d'acétate d'éthyle.

11. Le procédé selon les revendications 1 à 10, comprenant en outre la réaction dudit donepezil de formule (I) avec un acide pharmaceutiquement acceptable pour former un sel d'addition d'acide de donepezil.

12. Le procédé selon les revendications 1 à 11, comprenant en outre;
- sur ledit donepezil ou sur ladite solution contenant du donepezil la détermination de la présence d'un composé de formule (XII) 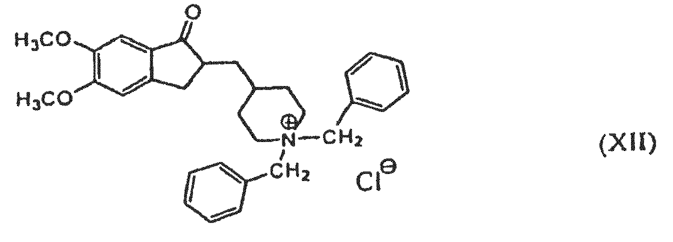 et
- si ledit composé de formule (XII) est présent en une quantité supérieure à un niveau prédétermine, à séparer alors ledit composé de formule (XII) dudit donepezil par extraction du donepezil à l'aide d'acétate d'éthyle.
